**Europäisches Patentamt**

**European Patent Office**

**Office européen des brevets**

(11) Numéro de publication : **0 334 774 B1**

# (12) FASCICULE DE BREVET EUROPEEN

(45) Date de publication du fascicule du brevet :
06.05.92 Bulletin 92/19

(51) Int. Cl.⁵ : **A61M 1/34,** B01D 61/00,
B01D 71/00

(21) Numéro de dépôt : **89420088.0**

(22) Date de dépôt : **10.03.89**

(54) **Dispositif intégré pour l'épuration biospécifique d'un liquide contenant des éléments cellulaires.**

(30) Priorité : **25.03.88 FR 8804234**

(43) Date de publication de la demande :
**27.09.89 Bulletin 89/39**

(45) Mention de la délivrance du brevet :
**06.05.92 Bulletin 92/19**

(84) Etats contractants désignés :
**AT BE CH DE ES FR GB IT LI LU NL SE**

(56) Documents cités :
DE-A- 2 828 549
GB-A- 2 040 724
US-A- 4 013 564

(73) Titulaire : **HOSPAL INDUSTRIE**
**7, Avenue Lionel Terray**
**F-69330 Meyzieu (FR)**

(72) Inventeur : **Pusineri, C.**
**33 rue des Fleurs Serezin du Rhône**
**F-69360 St Symphorien d'Ozon (FR)**
Inventeur : **Cronenberger, M.**
**Saint-Sorlin**
**F-69440 Mornant (FR)**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne le domaine technique du traitement de liquides biologiques pour les débarrasser de substances indésirables qui y sont contenues. Plus particulièrement, la présente invention concerne un dispositif pour la filtration et l'épuration biospécifique d'un liquide biologique, notamment du sang.

Il existe par exemple actuellement de nombreuses pathologies qui sont associées à la présence, dans le sang de composants indésirables. Il est donc intéressant de pouvoir débarrasser sélectivement le sang de ces substances, et de nombreux dispositifs ont été proposés à cette fin.

Ainsi la demande de brevet français 2 035 134 propose un dispositif permettant d'éliminer du sang des protéines ou des acides aminés déterminés par passage sur une substance active. Un tel dispositif présente cependant un risque de dégradation des éléments figurés du sang et notamment des érythrocytes. L'hémolyse consécutive au traitement du sang de cette manière est donc l'inconvénient majeur d'une telle technique.

Comme les substances indésirables que l'on souhaite éliminer du sang sont présentes dans la fraction liquide du sang que l'on appelle le plasma sanguin, il a été proposé dans l'art antérieur, notamment dans la demande de brevet français 2 325 390, d'effectuer l'épuration en traitant uniquement le plasma sanguin. Ainsi, cette demande de brevet propose un système de traitement du sang comportant un premier dispositif pour séparer le plasma des éléments figurés du sang, puis en série, un second dispositif de purification du plasma. Un tel système, s'il présente l'avantage de ne pas nécessiter de contact entre les éléments figurés du sang et l'agent de traitement contenu dans le dispositif de purification, présente cependant l'inconvénient de nécessiter un volume important de liquide en circulation. Or, ce volume de liquide est soustrait à la circulation sanguine du patient, il est donc très important de le minimiser le plus possible.

Il a donc été proposé dans l'art antérieur et notamment dans la demande EP 139 949 un dispositif comportant un faisceau interne de fibres creuses constituées par une membrane semi-perméable, destinées à séparer le plasma des éléments figurés du sang. Ce faisceau de fibres creuses est entouré par une chambre de traitement contenant un agent de traitement du plasma obtenu par filtration du sang circulant à l'intérieur des fibres creuses. Ce dispositif utilise les processus de filtration et de rétrofiltration, c'est-à-dire que dans la première partie du dispositif correspondant à l'entrée du sang à traiter, la pression du sang à l'intérieur de la fibre entraine un passage de plasma par convection de l'intérieur de la fibre vers la chambre de traitement. A l'inverse, dans la seconde partie, proche de la sortie du dispositif, la pression du sang à l'intérieur de la fibre étant plus faible que la pression du plasma dans la chambre de traitement, le plasma rétrofiltre pour rejoindre le sang enrichi en éléments figurés. Le plasma obtenu par filtration du sang à travers la membrane semi-perméable constituant la paroi des fibres creuses est traité par un agent contenu dans la chambre de traitement; ainsi, à la sortie de ce dispositif, on obtient une recombinaison des éléments figurés du sang avec le plasma épuré.

Si ce dispositif permet de pallier à un certain nombre d'inconvénients de l'art antérieur, il présente cependant le désavantage d'être limité par le débit de plasmafiltration à travers la membrane semi-perméable. En effet, pour pouvoir utiliser les processus de filtration et de rétrofiltration, la différence de pression de part et d'autre de la membrane de plasmaphérèse doit s'annuler au centre du module ce qui entraine alors à cet endroit un débit de filtration nul.

Pour augmenter le débit global de plasmafiltration, il faudrait pouvoir augmenter la surface de filtration, le débit du sang à traiter ou la perte de charge du sang à l'intérieur du dispositif. Etant donné sa capacité de filtration limitée, un tel dispositif ne permet donc pas de satisfaire aux exigences de performance actuelles.

Le brevet US 4 013 564 qui forme le préambule de la revendication 1 décrit également un dispositif d'épuration du sang comportant un agent de traitement sous forme de particules emprisonnées dans un espace limité par deux membranes. La fraction sanguine à épurer est séparée du sang grâce à une membrane microporeuse. Cette fraction est ensuite épurée par passage à travers les particules constituant l'agent de traitement et elle traverse ensuite le mambrane retenant l'agent de traitement.

Il a également été proposé dans le brevet US 4 361 484 un dispositif d'épuration du sang constitué d'une membrane microporeuse dans les pores et à la surface de laquelle des substances biologiques actives sont immobilisées, mais uniquement dans la partie de la membrane éloignée du passage du sang. Selon le texte de ce brevet, le dispositif d'épuration comporte en outre des moyens pour soumettre le sang à épurer à des variations de pression de façon à provoquer un passage alternatif d'une fraction du sang à travers les micropores de la membrane. Ainsi, seule la fraction plasmatique du sang à épurer pénètre à travers les micropores de la membrane et entre en contact avec les substances biologiquement actives. Un tel dispositif permet donc d'éviter la phase préliminaire de plasmafiltration tout en évitant le contact entre les cellules sanguines du sang et les substances biologiques actives. Cependant, outre le fait qu'il soit de mise en oeuvre complexe, un tel dispositif présente de sérieuses difficultés de fabrication. De plus, il semble difficile, avec un tel dispositif, d'atteindre un taux d'épuration suffisant.

Ainsi les dispositifs d'épuration biospécifique de l'art antérieur ne permettent pas d'effectuer le traitement d'un liquide biologique, notamment du sang, de façon satisfaisante.

L'objet de la présente invention est de proposer un dispositif d'épuration biospécifique ne présentant pas les inconvénients de l'art antérieur et permettant une bonne épuration d'un liquide biologique, notamment du sang, sans risque de destruction des cellules.

Un autre objet de la présente invention est de proposer un dispositif d'épuration biospécifique, ne nécessitant pas un important volume de liquide en circulation.

Un autre objet de la présente invention est de proposer un dispositif d'épuration biospécifique dont la fabrication peut être réalisée facilement et à des prix de revient peu importants.

Un autre objet de la présente invention est de proposer un dispositif d'épuration biospécifique présentant une bonne efficacité par rapport à la taille et à l'encombrement du dispositif.

Un autre objet de la présente invention est de proposer un dispositif d'épuration biospécifique dont la mise en oeuvre est simple, fiable et présente toutes les sécurités nécessaires pour le patient en cours de traitement.

Pour pallier aux inconvénients de l'art antérieur, la présente invention propose un dispositif pour l'épuration biospécifique d'un liquide contenant des éléments cellulaires, du type comprenant un boîtier muni de :

– au moins une canalisation d'entrée du liquide à traiter,
– au moins une canalisation de sortie du liquide concentré en éléments cellulaires,
– une canalisation de sortie de la fraction liquide épurée, et à l'intérieur duquel est disposée au moins une membrane pour séparer par filtration tangentielle la fraction liquide des éléments cellulaires du liquide à traiter,

caractérisé en ce que ledit boîtier comporte en outre au moins une membrane microporeuse pour l'épuration biospécifique par filtration transversale de la fraction liquide dudit liquide à traiter.

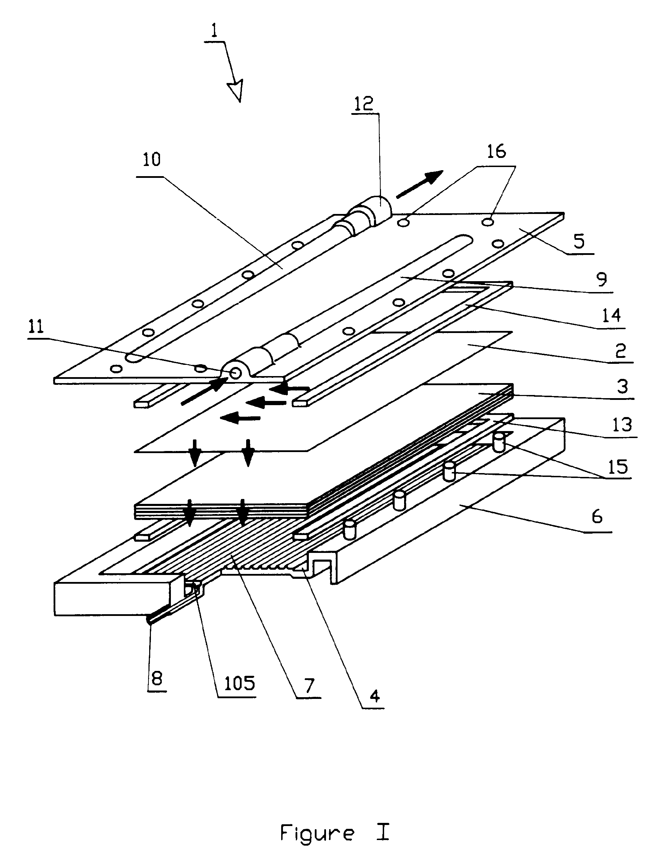

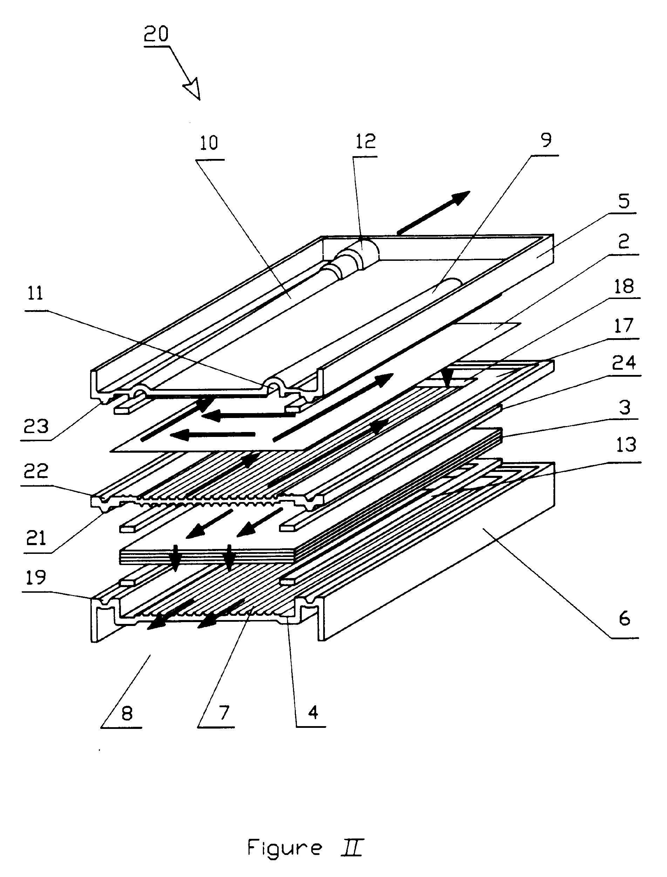

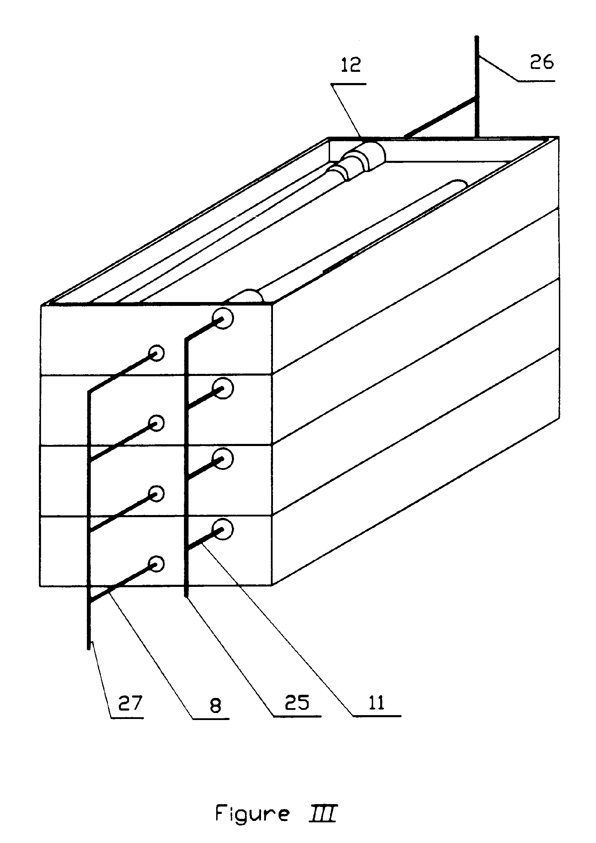

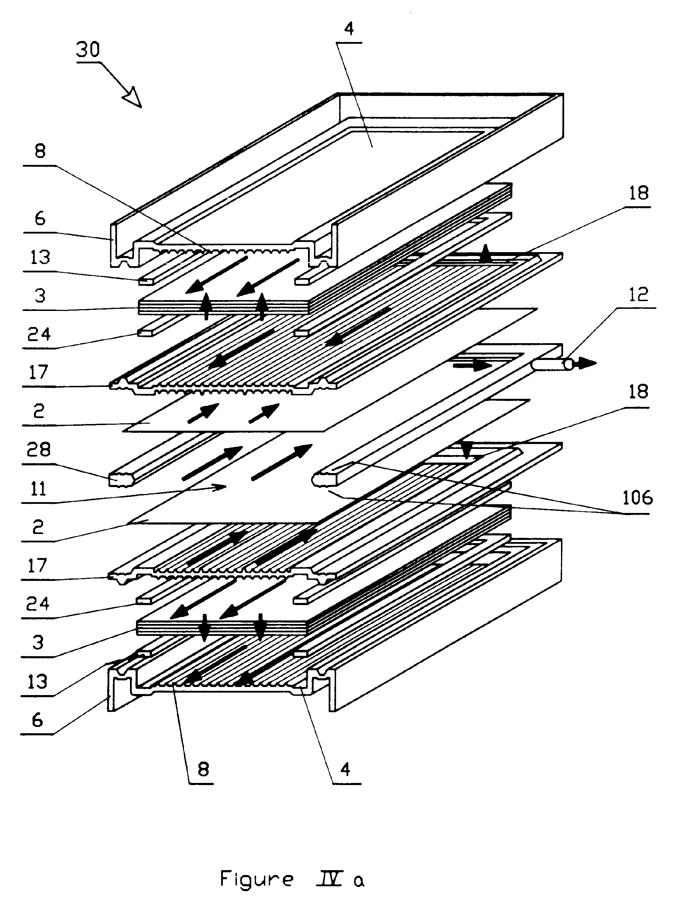

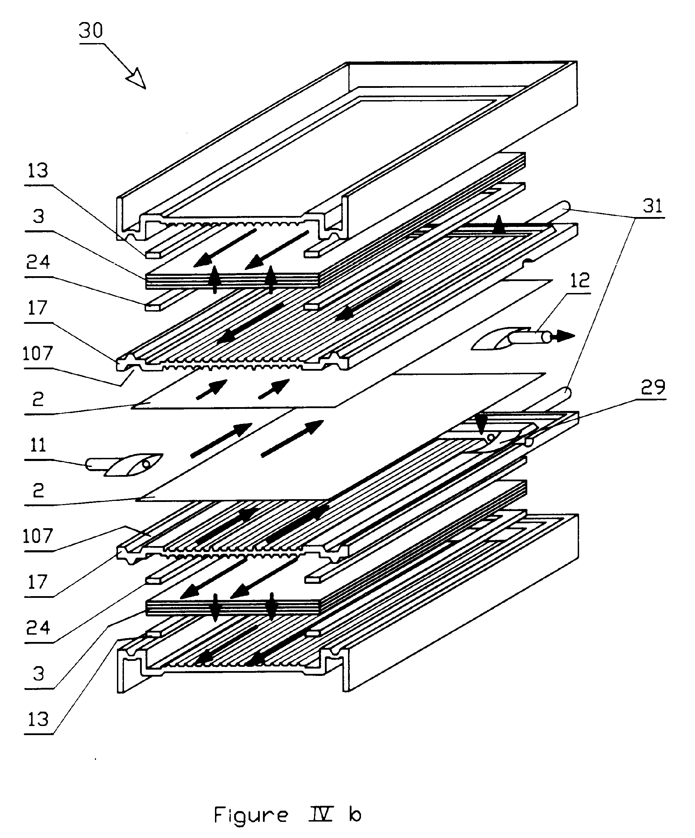

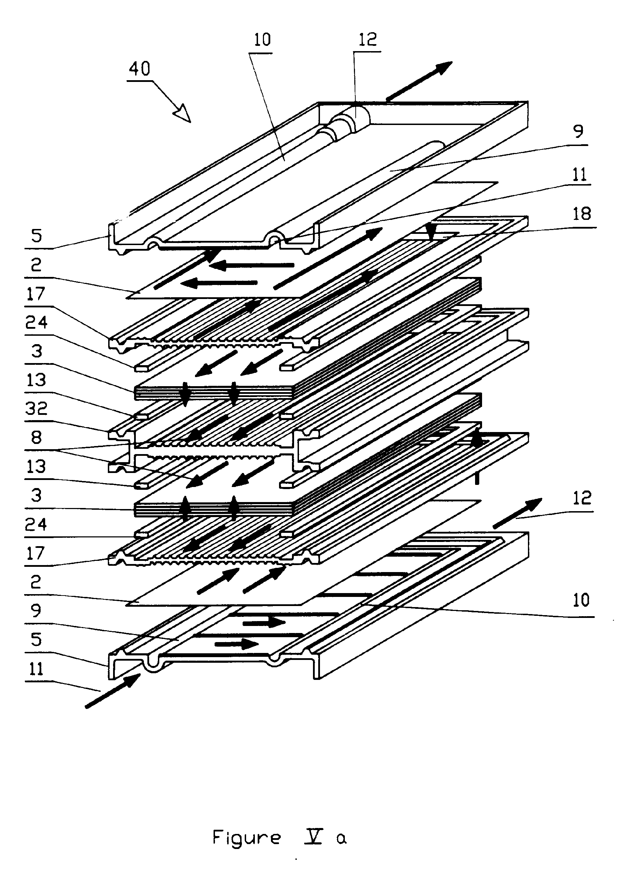

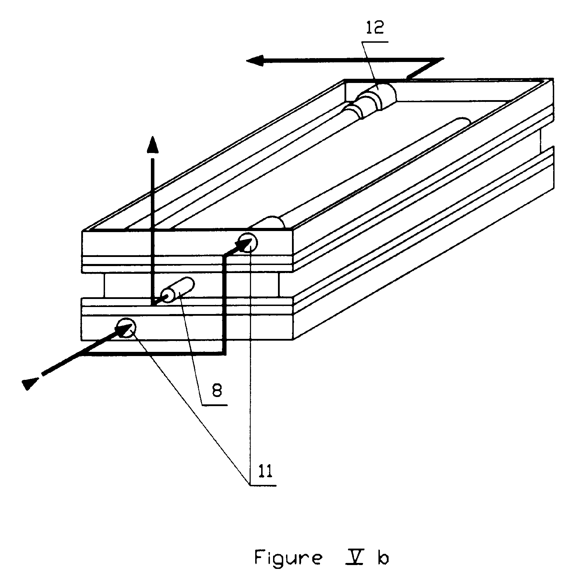

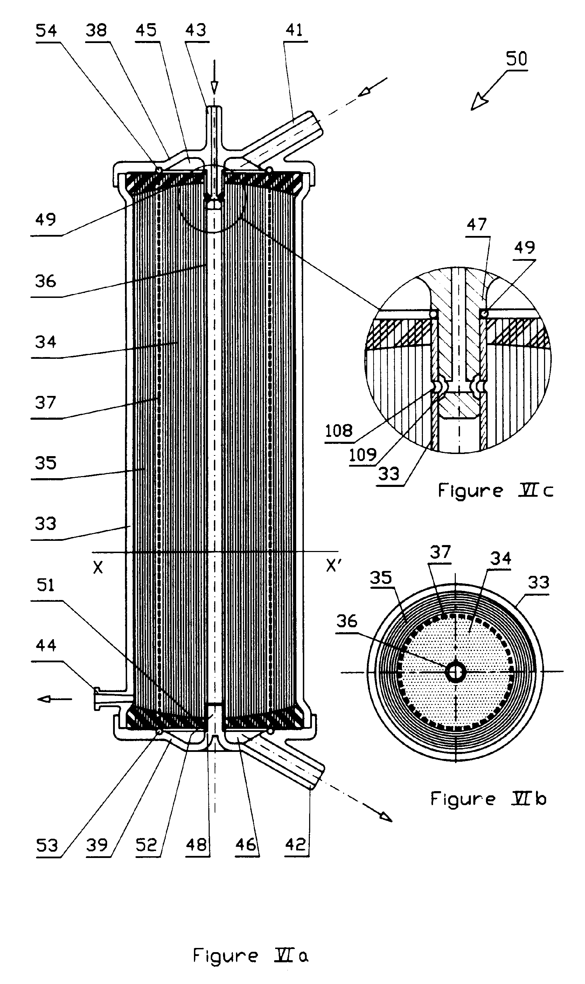

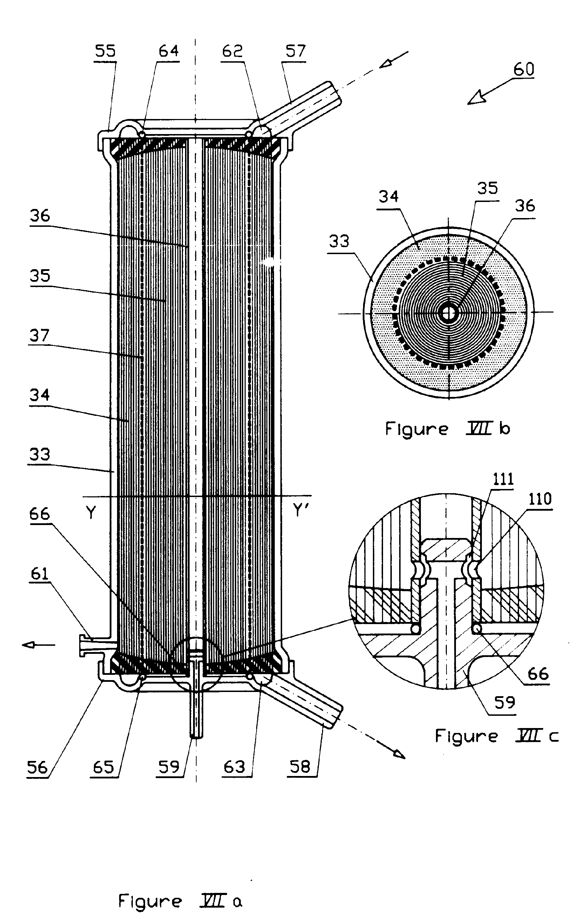

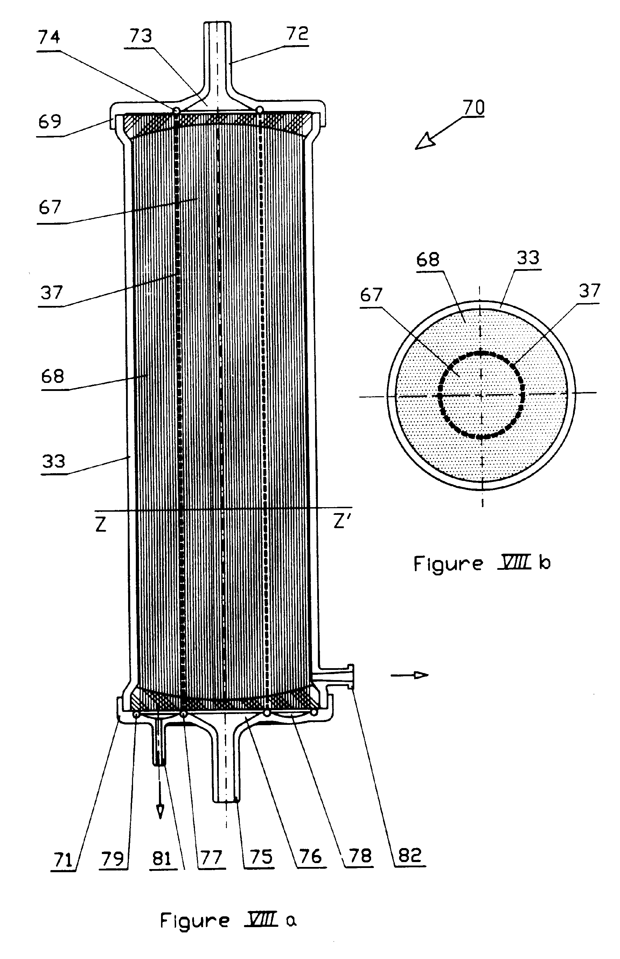

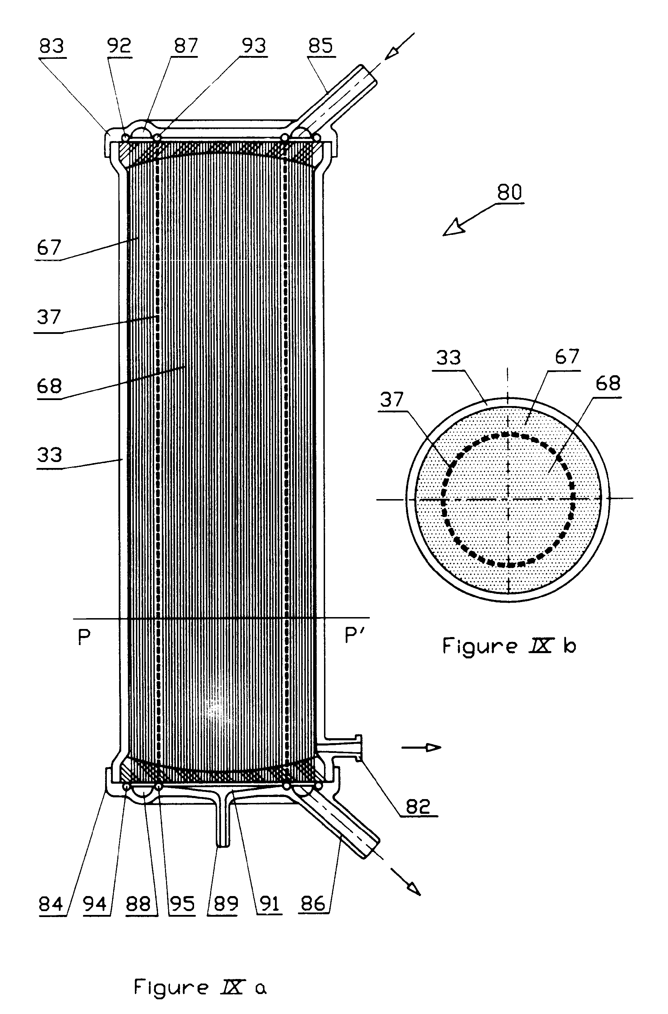

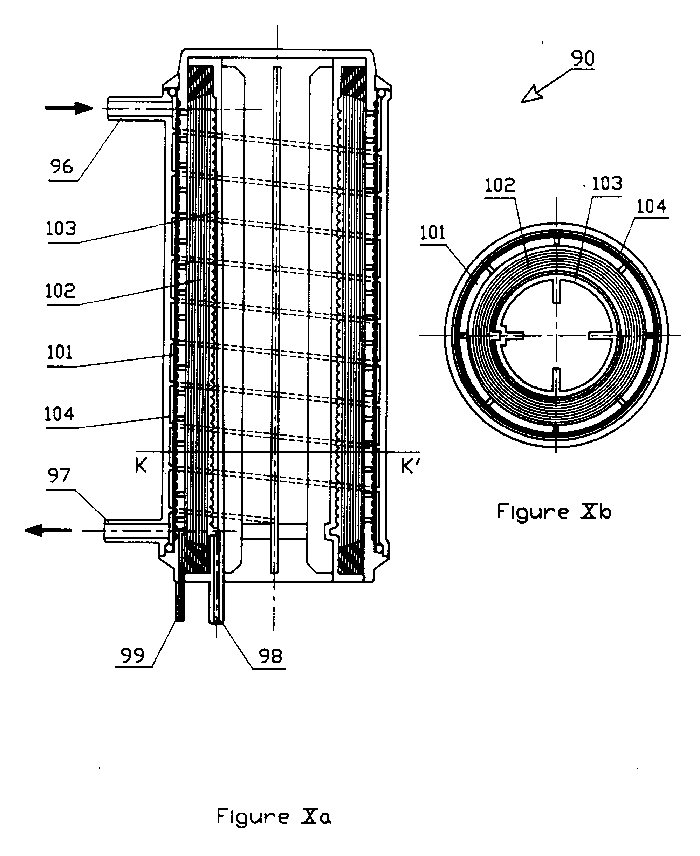

D'autres objets et avantages de la présente invention apparaîtront à la lecture de la description qui va suivre en référence aux dessins annexés qui illustrent à titre d'exemples et sans échelle déterminée, différents modes de réalisation de l'objet de la présente invention. La figure I est une vue éclatée d'un premier mode de réalisation du dispositif objet de la présente invention. La figure II représente de façon schématique et éclatée un second mode de réalisation de la présente invention. La figure III est une représentation en perspective d'un assemblage de dispositifs représentés sur les figures I ou II.

Les figures IVa et IVb sont des représentations éclatées d'un troisième mode de réalisation de la présente invention.

La figure Va est une représentation éclatée d'un quatrième mode de réalisation de la présente invention.

La figure Vb représente en perspective le dispositif de la figure Va assemblée.

La figure VIa est une coupe longitudinale d'un dispositif selon un cinquième mode de réalisation de la présente invention.

La figure VIb est une coupe transversale selon le plan XX' du dispositif selon le mode de réalisation de la figure VIa.

La figure VIIa est une coupe longitudinale d'un dispositif selon un sixième mode de réalisation de la présente invention.

La figure VIIb est une coupe transversale selon le plan YY' du dispositif selon le mode réalisation de la figure VIIb.

La figure VIIIa est une coupe longitudinale d'un dispositif selon un septième mode de réalisation de la présente invention.

La figure VIIIb est une coupe transversale selon le plan ZZ' du dispositif représenté sur la figure VIIIa.

La figure IXa est une coupe longitudinale d'un dispositif selon un huitième mode de réalisation de la présente invention.

La figure IXb est une coupe transversale selon le plan PP' du dispositif représenté sur la figure IXa.

La figure Xa est une coupe longitudinale d'un dispositif selon un neuvième mode de réalisation de la présente invention.

La figure Xb est une coupe transversale selon le plan KK' du dispositif représenté sur la figure Xa.

Pour simplifier, les éléments homologues des différentes figures conserveront les mêmes numéros de référence.

En ce référant à la figure I, on voit que le dispositif d'épuration représenté dans son ensemble par la référence 1 est essentiellement constitué par une membrane plane 2 de filtration du liquide à traiter, et par une structure microporeuse 3 constituée par un empilement de membranes planes d'épuration biospécifique. Cet ensemble de membranes est disposé à l'intérieur d'une enceinte constitué essentiellement par une plaque inférieure 4 et une plaque supérieure 5.

En effet, la palque inférieure 4, présente, en périphérie, une surélévation 6 constituant les parois latérales du dispositif d'épuration 1.

La face interne de cette plaque inférieure 4 est en outre munie de cannelures longitudinales ou de tout autre réseau approprié de annelures, constitué par exemple par des cannelures sinusoïdales ou en zig-zags continues ou discontinues, destinées à favoriser la collecte et la circulation du liquide obtenu après filtration à travers l'ensemble des membranes. Ce réseau de cannelures définit une zone utile 7 pour la circulation du liquide épuré.

A l'extrémité de la zône utile 7 une canalisation transversale 105 permet de recueillir le liquide épuré.

La plaque inférieure 4 est également pourvue d'une canalisation 8 de communication avec l'extérieur constituant la sortie du liquide épuré ; cette canalisation 8 est avantageusement formée dans la paroi latérale 6 à l'une des extrémités de la zone utile 7.

La plaque supérieure 5 est munie de deux canalisations latérales 9 et 10 s'étendant longitudinalement et de façon sensiblement parallèle ; ces canalisations 9 et 10 sont destinées respectivement à la distribution du liquide à traiter et à la collecte du liquide concentré en éléments cellulaires ; elles communiquent avec l'extérieur par des ouvertures constituant, d'une part l'entrée 11 du liquide à traiter et d'autre part, la sortie 12 du liquide concentré en éléments cellulaires. Avantageusement, l'ouverture 11 est située sur un bord de la plaque 5 opposé au bord sur lequel est située l'ouverture 12. Cette disposition permet d'assurer une bonne circulation du liquide à traiter en évitant la formation de zone de stagnation appelée "zone morte".

En outre, le profil des canalisations 9 et 10 est tel que leur section décroit de façon sensiblement constante à partir des ouvertures 11 et 12. Ceci a pour but de favoriser la distribution du liquide à traiter en maintenant une vitesse sensiblement constante tout au long de chacune des canalisations.

Afin que le liquide à traiter se répartisse entre la canalisation 9 et la canalisation 10 selon un film d'épaisseur sensiblement constante, la plaque supérieure 5 est munie sur sa face interne de nervures ou butées (non représentées) disposées à intervalles réguliers. Grâce à un serrage à pression constante lors de l'assemblage du dispositif, ces nervures ou butées permettent d'assurer, entre la plaque supérieure 5 et la membrane de filtration 2, un écartement sensiblement constant sur toute la surface de la membrane.

La membrane de filtration 2 est une membrane plane permettant de séparer, par filtration tangentielle, les éléments cellulaires du liquide à épurer.

Dans le cas où le liquide à épurer est du sang, cette membrane de filtration 2 est par exemple, une membrane de plasmaphérèse assurant la séparation du plasma des cellules du sang. Un exemple de membrane convenant particulièrement bien est la membrane fabriquée par RHONE-POULENC portant la dénomination TPC 19 et dont la méthode de fabrication est décrite dans le demande de brevet français enregistrée sous le no 82/00485.

La structure microporeuse 3 d'épuration biospécifique est constitué par exemple par un ensemble de membranes planes microporeuses traitées de façon appropriée pour assurer l'épuration biospécifique du liquide obtenu par filtration tangentielle le long de la membrane 2. Il s'agit de membranes présentant des groupements chimiques réactifs permettant le greffage de molécules épuratrices, constituées par exemple par des protéines, des glycoprotéines, des polysaccharides ou de façon plus générale par des dérivés organiques d'origine naturelle, synthétiques ou semi-synthétiques présentant une affinité biospécifique pour les dérivés à épurer.

On peut par exemple utiliser les membranes commercialisées sous la dénomination Biodyne Immuno-Affinity par la Société Pall, sur lesquelles auront été greffés par fixation covalente directe des anticorps choisis pour leur capacité à reconnaitre et à se lier sélectivement avec des substances qui leur sont complémentaires et que l'on souhaite éliminer du liquide à épurer.

Il est également possible d'utiliser la membrane commercialisée par la société Millipore, sous la dénomination Immobilon sur laquelle auront été fixées par liaisons covalentes les protéines spécifiques telles des anticorps, capables de réagir sélectivement avec les substances à épurer.

Afin d'obtenir une surface de traitement suffisante pour tout le liquide à épurer, il est possible d'empiler plusieurs membranes planes les unes sur les autres.

Au lieu d'utiliser un empilement de membranes planes, il serait également possible d'utiliser une seule membrane microporeuse dont l'épaisseur permettrait d'obtenir une surface de traitement équivalente à celle que représente l'ensemble des membranes empilées.

La structure microporeuse nécessaire pour assurer l'épuration biospécifique peut également être obtenue par tout moyen approprié, par exemple en utilisant une nappe de matériau non-tissé avec un taux de vide important ou par compaction de fibres ou de billes, ou même par assemblage de fils.

L'agencement des différents éléments constituant le dispositif d'épuration 1 est le suivant.

La structure microporeuse 3 constituée par l'empilement de membranes d'épuration est disposée sur la plaque inférieure 4 à l'intérieur de l'enceinte limitée par les bords surélevés 6. L'étanchéité entre la plaque inférieure 4 et cet empilement est assurée par un joint-cadre 13.

On dispose sur cet empilement de membranes d'épuration une membrane de filtration 2 destinée à séparer par filtration tangentielle les éléments cellulaires du liquide à épurer, tels que, par exemple, les cellules sanguines du plasma dans le cas où le liquide à épurer est du sang. L'étanchéité de cette membrane avec la plaque supérieure 5 est assurée par un joint-cadre 14, qui est avantageusement du même type que le joint 13.

Ces joints peuvent être remplacés par tout moyen équivalent tel qu'un empotage périphérique des membranes avec, par exemple, une colle en polyuréthanne.

Afin d'assurer l'assemblage du dispositif, des tétons d'assemblage 15 sont répartis sur les bords surélevés 6 de la plaque inférieure 4. Ces tétons 15 sont destinés à s'introduire dans les trous correspondants 16 situés en périphérie de la plaque supérieure 5. On pourra ainsi effectuer une fixation du dispositif d'épuration par rivetage par ultrasons ou par thermoformage par exemple.

Le dispositif d'épuration 1 permet ainsi d'intégrer dans un seul boitier une membrane de filtration du liquide à épurer ainsi qu'une structure microporeuse d'épuration biospécifique.

Le fonctionnement du dispositif d'épuration 1 est le suivant. Le liquide à traiter est introduit dans la canalisation 9 par l'entrée 11. Cette canalisation 9 permet de répartir le liquide à traiter sur toute la longueur de la plaque supérieure 5. Ce liquide se répartit ensuite sous la forme d'un film entre la canalisation d'entrée 9 et la canalisation de sortie 10. Le sens de circulation du liquide est indiqué sur la figure I par des flèches. Cette circulation du liquide à traiter sous forme de film permet de séparer certains éléments du liquide par filtration tangentielle le long de la membrane de filtration 2. Dans le cas où liquide à épurer est du sang, la filtration tangentielle du sang le long d'une membrane de plasmaphérèse 2 permet de séparer le plasma sanguin qui traverse la membrane des éléments cellulaires qui, en raison de leur taille, sont retenues et sont évacués à l'extérieur du dispositif par la canalisation 10 et la sortie 12.

Le liquide obtenu après filtration à travers la membrane 2 est ensuite filtré, mais dans ce pas par filtration transversale, au travers de l'empilement de membranes d'épuration, c'est-à-dire que tout le liquide à épurer traverse la structure microporeuse que représente l'empilement de membranes d'épuration.

Les molécules épuratrices greffées à la surface et dans les micropores des membranes d'épuration retiennent sélectivement, lors du passage transversal du liquide à épurer, les molécules que l'on souhaite éliminer. Le liquide ainsi épuré est recueilli sur la plaque inférieure 4 grâce au réseau de cannelures 7 avant d'être évacué à l'extérieur par la sortie 8.

Dans le cas où le liquide à épurer est du sang, le plasma obtenu après filtration tangentielle le long de la membrane 2 est épuré par filtration transversale à travers les membranes d'épuration. Le plasma épuré est évacué à l'extérieur du dispositif par la sortie 8. Ce plasma peut éventuellement être recombiné avec les éléments cellulaires provenant de la sortie 12, pour être ensuite retourné au patient en cours de traitement, si le sang à traiter provient de la circulation extracorporelle d'un patient.

Ainsi, grâce au dispositif 1 selon l'invention, on peut intégrer dans un seul boitier le traitement d'un liquide tout d'abord par filtration tangentielle puis aussitôt par filtration transversale.

Selon le mode de réalisation représenté à la figure II, on voit que le dispositif d'épuration désigné dans son ensemble par la référence 20 comporte entre la membrane de filtration 2 et la structure microporeuse 3 une plaque intermédiaire 17. Cette plaque intermédiaire constitue un support pour la membrane de filtration 2, et permet donc d'assurer un écartement sensiblement constant entre la plaque supérieure 5 et la membrane de filtration 2, même dans le cas où on effectue l'assemblage du dispositif à cote constante, ce qui d'un point de vue pratique est préférable à un assemblage à pression constante.

Ainsi, le film de liquide à traiter qui s'écoule entre la canalisation d'entrée 9 et la canalisation de sortie 10 présente une épaisseur sensiblement constante sur toute sa surface. La plaque intermédiaire 17 est munie sur chacune de ses deux faces principales, de cannelures, définissant, de la même façon que le réseau de cannelures de la face interne de la plaque inférieure 4, une zone utile pour la circulation du liquide filtré à épurer. A l'une des extrémités de cette zone utile, préférentiellement, à l'extrémité opposée à l'entrée du liquide à traiter, il est prévu dans l'épaisseur de la plaque, un passage 18 permettant au liquide filtré recueilli sur l'une des faces de la plaque intermédiaire 17 de se répartir le long de l'autre face de la plaque avant d'être épuré par filtration transversale à travers la structure microporeuse 3 constituée par l'empilement de membranes microporeuses. Il est également possible de prévoir une plaque intermédiaire 17 avec des trous répartis sur toute sa surface. A lieu d'emprunter le passage 18, le liquide à épurer traverserait directement la plaque 17 pour être ensuite traité par filtration à travers les membranes d'épuration.

Afin d'assurer l'assemblage du dispositif, la surélévation 6 de la plaque inférieure 4 est munie, non pas de tétons d'assemblage 15 comme le dispositif d'épuration 1 mais d'une rainure périphérique 19 destinée à recevoir une saillie correspondante 21 située sur la périphérie de la face inférieure de la plaque intermédiaire 17. De façon similaire, la plaque intermédiaire 17 possède sur sa face supérieure une rainure périphérique 22 destinée à recevoir une saillie correspondante 23 située sur la périphérie de la face inférieure de la plaque inférieure 5.

La liaison entre saillie et rainure peut être effectuée par collage ou par soudage, par exemple thermique ou aux ultra-sons. On peut également effectuer l'assemblage du dispositif par tout autre moyen approprié, par exemple en utilisant des plaques latérales (non représentées) munies de rebords en queue d'aronde qui maintiendraient en contact l'ensemble des éléments du dispositif.

Les joints-plans périphériques 13 et 24 situés de part et d'autre de l'empilement de membranes 3 sont destinés à la fois à assurer l'étanchéité du dispositif 20 et à maintenir un espace suffisant pour la circulation du liquide.

Le fonctionnement du dispositif d'épuration 20 tel que représenté sur la figure II est le suivant. Les trajets d'écoulement sont symbolisés par les flèches. Le liquide à traiter est introduit par la canalisation 9, à partir de laquelle il s'écoule latéralement pour atteindre la canalisation de sortie 10. Cette circulation du liquide permet, de la même façon que dans le dispositif d'épuration 1 représenté sur la figure I, d'assurer la séparation des éléments cellulaires du liquide à épurer par filtration tangentielle le long de la membrane de filtration 2.

La fraction liquide ainsi obtenue est recueillie dans les cannelures situées sur la face supérieure de la plaque intermédiaire 17 et s'écoule jusqu'au passage 18 situé à l'extrémité de la plaque. Le liquide à épurer emprunte ce passage 18 pour s'écouler ensuite dans les cannelures situées sur la face inférieure de cette plaque intermédiaire 17.

Le liquide ainsi réparti s'écoule ensuite par filtration transversale à travers l'empilement de membranes microporeuses traitées pour l'épuration biospécifique.

Le liquide épuré est ensuite recueilli grâce aux nervures de la plaque inférieure 4, dans la canalisation 105 avant d'être évacué par la sortie 8.

Dans le cas où les surfaces de traitement que représentent la membrane de filtration 2 et la structure microporeuse 3 ne sont pas suffisantes, il est possible, ainsi que cela est représenté sur la figure III, de placer plusieurs dispositifs d'épuration en parallèle.

Dans ce cas, les entrées 11 du liquide à épurer sont réunies en une canalisation 25 permettant d'introduire le liquide à traiter ; de même, les sorties 12 du liquide concentré en éléments cellulaires et les sorties 8 du liquide épuré sont réunies respectivement en une canalisation 26 et en une canalisation 27.

On peut ainsi, grâce à cette association en parallèle de plusieurs dispositifs multiplier la capacité de traitement d'un liquide.

Dans le mode de réalisation représenté sur la figure IVa le dispositif d'épuration 30 est un dispositif à membrane plane du type bimembranaire. Ce dispositif 30 comporte en effet une seule entrée 11 du liquide à traiter, une seule sortie 12 du liquide concentré en éléments cellulaires, mais deux sorties 8 du liquide épuré.

Ce dispositif est constitué de deux unités d'épuration du liquide à traiter comportant chacune une membrane de filtration 2, une plaque intermédiaire 17 ainsi qu'une structure microporeuse 3 constituée par un empilement de membranes microporeuses traitées pour l'épuration biospécifique du liquide filtré.

Les deux unités d'épuration sont disposées symétriquement par rapport à un cadre médian 28 séparant les deux membranes de filtration 2. Ce cadre médian 28 est muni de godron 106 destinés à faire l'étanchéité avec les bords périphériques des cadres médians 17.

Le liquide à traiter est introduit dans le dispositif d'épuration par une entrée 11 située sur le cadre médian à une des extrémités du dispositif. Il s'écoule alors entre les deux membranes où il est filtré. Le liquide concentré en éléments cellulaires est recueilli à l'extrémité opposée du dispositif et est évacué par une sortie 12 située sur le cadre médian 28.

La filtration tangentielle du liquide le long de chacune des membranes de filtration 2 permet de recueillir du liquide filtré dépourvu d'éléments cellulaires le long de l'une des faces de chacune des plaques intermédiaires 17. Ce liquide filtré s'écoule ensuite par les passages 18 pour se répartir ensuite le long de l'autre face de chacune des plaques intermédiaires avant d'être épuré par filtration transversale au travers de la structure microporeuse 3.

La filtration et l'épuration du liquide à traiter s'effectue ainsi de façon symétrique dans chacune des unités d'épuration.

Le dispositif d'épuration 30 permet sous une forme très compacte, d'augmenter sensiblement les capacités de filtration et d'épuration du dispositif 20 représenté sur la figure II.

Selon le mode de réalisation représenté sur la figure IVb, le cadre médian 28 du dispositif d'épuration 30 est supprimé ; l'entrée 11 du liquide à traiter et la sortie 12 du liquide concentré en éléments cellulaires sont constitués par deux embouts logés entre les plaques intermédiaires 17 des deux unités d'épuration grâce à des évidements 29 prévus à cet effet. L'étanchéité est assurée au niveau des membranes 2 de séparation des éléments cellulaires du liquide à traiter grâce à un remplissage des gorges 107 des plaques intermédiaires 17.

De façon avantageuse, le dispositif 30 comporte en outre sur chacune des plaques intermédiaires 17 des prises de pression 31 du plasma à épurer.

Selon le mode de réalisation représenté sur les figures Va et Vb, le dispositif d'épuration 40 est également un dispositif à membranes planes du type bimembranaire. Ce dispositif 40 est constitué de deux unités d'épuration de la même façon que le dispositif 30, mais avec dans ce cas-ci deux entrées 11 du liquide à traiter, deux sorties 12 du liquide enrichi en éléments cellulaires, une sortie unique 8 du liquide épuré. Les deux unités d'épuration comportent chacune une plaque externe 5 du même type que la plaque supérieure 5 du dispositif 1 représenté sur la figure I et comportant deux canalisations latérales 9 et 10 respectivement d'entrée et de sortie du liquide. Chaque unité d'épuration comprend également une membrane 2 de séparation des éléments cellulaires par filtration tangentielle, une plaque intermédiaire 17, une structure microporeuse 3 constituée par un empilement de membranes microporeuses traitées pour l'épuration biospécifique du liquide à traiter.

La structure du dispositif 40 est telle que les divers éléments constituant chacune des unités d'épuration sont assemblés de façon symétrique par rapport à une plaque centrale 32 séparant les deux empilements de membranes traitées pour l'épuration biospécifique. Cette plaque centrale comporte sur chacune de ses faces des cannelures longitudinales formant une zone utile pour la circulation du liquide épuré.

Les écoulements à l'intérieur du dispositif 40 sont symbolisés par les flèches représentées sur la figure Va. Le liquide à traiter est introduit par chacune des canalisations 9 des plaques 5. La séparation des éléments cellulaires du liquide à traiter est effectuée par filtration tangentielle le long des membranes de filtration 2.

Le liquide concentré en éléments cellulaires est recueilli dans la canalisation 10 à partir de laquelle il est évacué à l'extérieur du dispositif. Le liquide obtenu par filtration le long de la membrane 2 s'écoule sur la zone utile de l'une des faces de la plaque intermédiaire pour emprunter le passage 18 avant de se répartir sur l'autre face de la plaque 17. Ce liquide à épurer traverse ensuite la structure microporeuse 3 constituée par l'empilement de membranes d'épuration. On recueille alors le liquide épuré grâce aux cannelures situées sur chacune des faces de la plaque centrale 32. L'ensemble du liquide épuré ainsi obtenu est conduit à l'extérieur du dispositif 40 par la sortie 8. Dans le cas où le liquide à épurer est du sang, on obtient du plasma par filtration tangentielle le long de la membrane de plasmafiltration 2. Ce plasma est épuré par filtration transversale au travers des membranes d'épuration. Le plasma épuré recueilli grâce aux cannelures de la plaque centrale est conduit à l'extérieur par la sortie 8 ; ce plasma épuré peut ensuite être recombiné avec le sang concentré en éléments cellulaires provenant de la canalisation de sortie 10 avant d'être, par exemple, renvoyé à un patient d'où provient le sang à épurer.

Selon le mode préféré de réalisation représenté sur les figures VIa, VIb et VIc, le dispositif d'épuration 50 est un dispositif d'un type mixte comportant une membrane de type plan, ainsi que des fibres creuses. Ce dispositif d'épuration comprend dans une enveloppe cylindrique 33 une membrane de filtration 34 constituée par un faisceau interne de fibres creuses ainsi qu'une membrane 35 plane microporeuse traitée pour l'épuration biospécifique du liquide. Le faisceau de fibres creuses entoure un tube central 36, et la membrane d'épuration 35 est elle-même enroulée autour du faisceau 34. Le tube central d'enroulement 36 peut être constitué par exemple en polycarbonate, en chlorure de polyvinyle ou en acrylonitrile-butadiène-styrène.

Les fibres creuses ainsi que la membrane plane enroulée sont scellées à chacune des extrémités du faisceau grâce à une résine d'empotage commune, en polyuréthane par exemple.

Afin de faciliter l'opération d'empotage et le maintien du faisceau de fibres creuses 34, on peut prévoir une grille intercalaire 37 entre le faisceau de fibres creuses 34 et la membrane d'épuration 35. Cette grille est par exemple constituée par un tissu ayant une épaisseur d'environ 100 à 110 microns en polyester monobrin. Le tissu commercialisé par la société UGB sous la référence mono TI 75-55775 convient tout à fait.

Il est possible également de placer une grille (non représentée) autour de l'ensemble constitué par le faisceau de fibres creuses et la membrane, afin de faciliter le recueil du liquide épuré.

Ainsi, que cela apparait clairement sur la figure VIb, l'enroulement de la membrane 35 est effectuée de façon continue et ininterrompue. Afin d'éviter un écoulement éventuellement en spirale du liquide le long de la membrane 35 et de privilégier au contraire le passage transversal du liquide au travers notamment des micropores, il est possible à certaines étapes de l'enroulement, de coller la membrane selon une génératrice sur la couche précédente.

On peut aussi faciliter l'opération d'empotage ainsi que l'enroulement de la membrane d'épuration en adjoignant à cette dernière une fine grille jointive.

Le dispositif d'épuration 50 comporte à chacune des extrémités de l'enveloppe cylindrique 33 un couvercle (38, 39). Le couvercle 38 est muni d'une tubulure 41 destinée à l'entrée du liquide à traiter. Le profil interne de ce couvercle 38 est tel qu'il définit une chambre annulaire 45 de distribution du liquide à traiter dans laquelle débouche la tubulure 41. Ainsi que le montre la représentation détaillée de la figure VIc, la partie axiale du couvercle vient s'insérer légèrement à l'intérieur du faisceau de fibres creuses grâce à un épaulement 47, alors qu'elle se prolonge à l'extérieur par une tubulure 43 qui peut être reliée à des moyens (non représentés) de mesure de la pression du liquide filtré à épurer. Cette prise de pression est possible grâce à des ouvertures 108 dans le tube central 36 qui sont mises en communication avec la tubulure 43 par l'intermédiaire d'une gorge périphérique 109. L'étanchéité de la chambre de distribution 45 est assurée par deux joints toriques, respectivement 49 et 54.

Le couvercle 39 est muni d'une tubulure 42 destinée à la sortie du liquide concentré en éléments cellulaires. Le profil interne de ce couvercle 39 permet de définir une chambre annulaire 46 de recueil du liquide concentré en éléments cellulaires sur laquelle s'ouvre la tubulure 42. La partie axiale 52 du couvercle 39 vient s'insérer légèrement à l'intérieur du faisceau de fibres creuses grâce à un épaulement 48. L'étanchéité de la chambre de recueil est assurée par deux joints toriques, respectivement 51 et 53.

L'enveloppe cylindrique 33 comporte également une tubulure 44 permettant l'évacuation du liquide épuré.

Selon ce mode de réalisation, le liquide à traiter contenant des éléments cellulaires est introduit par la tubulure 41 dans la zone de distribution 45 du couvercle 38. Il pénètre alors à l'intérieur des fibres creuses 34 qui sont ouvertes sur la zone de distribution 45. La circulation du liquide à traiter à l'intérieur des fibres creuses permet de séparer, par filtration tangentielle, les éléments cellulaires du liquide à traiter.

Dans le cas où le liquide à traiter est du sang, on obtient ainsi une séparation des cellules sanguines du plasma qui est ensuite épuré par filtration transversale à travers la membrane plane microporeuse 35 traitée pour assurer l'épuration biospécifique du liquide dépourvu d'éléments cellulaires.

Le liquide concentré en éléments cellulaires est recueilli dans la zone de recueil 46 du couvercle 39 et est évacué à l'extérieur par la tubulure 42.

Le liquide épuré, qui dans le cas de traitement du sang est du plasma épuré, est évacué à l'extérieur du dispositif par la tubulure 44.

Ce plasma épuré pourra éventuellement être recombiné, à l'extérieur du dispositif d'épuration 50, avec le sang concentré en éléments cellulaires provenant de la tubulure 42, afin de reconstituer un sang complet mais épuré.

Selon le mode de réalisation représenté sur les figures VIIa, VIIb et VIIc, les fibres creuses 34 pour la filtration tangentielle du liquide à traiter sont situées en périphérie du dispositif d'épuration 60, alors que la membrane 35 traitée pour l'épuration biospécifique est enroulée autour du tube 36.

Dans ce cas, il est souhaitable de prévoir un grille interne (non représentée) autour du tube d'enroulement 36, ou des canaux d'écoulement sur le tube 36, afin de faciliter la circulation du liquide épuré.

Le couvercle 55 est munie d'une tubulure 57 d'entrée du liquide à traiter, mais dans ce cas, la zone de distribution 62 du liquide à traiter est située en périphérie du couvercle 55. Un joint torique 64 assure l'étanchéité de la zone de distribution 62 au niveau de la grille intercalaire 37.

Le couvercle 56 est munie d'une tubulure 58 de sortie du liquide concentré en éléments cellulaires et dans sa partie axiale, d'une tubulure 59 de sortie du liquide épuré ainsi que cela est représentée de façon détaillée sur la figure VIIc. Le liquide de dialyse épuré est recueilli dans la canalisation de la tubulure 59 grâce à une gorge périphérique 111 située sur la partie du couvercle venant s'insérer à l'intérieur du tube d'enroulement 36 et à des ouvertues 110 ménagées dans le tube 36 au niveau de la gorge périphérique. L'étanchéité entre la membrane 35 et la tubulure 59 est assurée par un joint torique 66. La zone de recueil 63 du liquide concentré en éléments cellulaires est située en périphérie du couvercle 56. L'étanchéité est assurée par un joint torique 65. L'enveloppe cylindrique 33 comporte également une tubulure 61 permettant une prise de pression du liquide filtré à épurer.

Selon le mode de réalisation représenté sur les figures VIIIa et VIIIb, le dispositif d'épuration 70 est un dispositif entièrement constitué de fibres creuses, notamment d'un faisceau interne 67 de fibres pour la filtration tangentielle du liquide à épurer et d'un faisceau externe 68 de fibres traitées pour l'épuration biospécifique. Les deux faisceaux sont séparés par une grille intercalaire 37 et sont disposés à l'intérieur d'une enveloppe cylindrique 33.

L'ensemble des fibres creuses est empoté à chacune des extrémités du dispositif grâce à une colle en polyuréthane par exemple. Le dispositif 70 comporte, de la même façon que les dispositifs 50 et 60, deux couvercles 69 et 71.

Le couvercle 69 est muni d'une tubulure axiale 72 destinée à l'entrée du liquide à traiter. Le profil du couvercle 69 est tel qu'une zone de distribution 73 du liquide à traiter communique avec l'intérieur des fibres creuses 67 situées au centre du dispositif. Un joint torique 74 assure l'étanchéité de la zone de distribution 73 au niveau de la grille intercalaire 37.

Le couvercle 71 est muni d'une tubulure axiale 75 destinée à la sortie du liquide concentré en éléments cellulaires, et sur sa périphérie, d'une tubulure 81 destinée à l'évacuation du liquide épurée. Le profil interne du couvercle 71 est tel qu'il forme une zone centrale de recueil 76 du liquide enrichi en éléments cellulaires, dans laquelle s'ouvre l'intérieur des fibres creuses 67 du faisceau interne. En outre, une zone de recueil annulaire 78 du liquide épuré est formée par une gorge périphérique dessinée sur la face interne du couvercle 71. L'étanchéité de cette zone de recueil est assurée par deux joints toriques 77 et 79.

L'enveloppe périphérique 33 comporte également une tubulure 82 permettant une prise de pression du liquide filtré à épurer. Le fonctionnement du dispositif d'épuration 70 selon ce mode de réalisation est le suivant.

Le liquide à traiter est introduit dans la zone de distribution 73 par la tubulure 72, puis pénètre à l'intérieur des fibres creuses constituant le faisceau interne 67. La circulation du liquide à l'intérieur de ces fibres 67 permet d'effectuer une séparation des éléments cellulaires du liquide par filtration tangentielle le long de la paroi des fibres. Le liquide enrichi en éléments cellulaires est recueilli dans la zone 76 puis évacué à l'extérieur par la tubulure 75.

La fraction filtrée du liquide à traiter traverse la grille intercalaire 37 et pénètre à l'intérieur des fibres creuses 68 par filtration à travers la membrane microporeuse constituant la paroi des fibres creuses 68. Cette membrane étant traitée pour assurer l'épuration biospécifique d'un liquide, le liquide circulant à l'intérieur de ces fibres creuses est un liquide épuré qui est recueilli dans la zone annulaire 78 et est évacué à l'extérieur par la tubulure 81.

Le dispositif d'épuration 80 représenté sur les figures IXa et IXb est également un dispositif constitué uniquement de fibres creuses.

Dans ce cas, les fibres creuses 67 pour la séparation des éléments cellulaires du liquide à épurer par filtration tangentielle sont situées à la périphérie du dispositif, alors que les fibres creuses 68 dont la paroi constitue la structure microporeuse pour l'épuration biospécifique, forment la partie interne du faisceau. Les deux faisceaux sont séparés par une grille intercalaire 37

Le couvercle 83 est muni sur sa face interne d'une gorge périphérique constituant une zone annulaire 87 de distribution du liquide à traiter.

L'étanchéité de cette zone de distribution est assurée par deux joints toriques 92 et 93.

Le couvercle 84 est muni sur sa face interne d'une gorge périphérique constituant une zone de recueil 88 du liquide concentré en éléments cellulaires, dans laquelle débouche une tubulure 86 d'évacuation de ce liquide concentré. L'étanchéité de cette zone de recueil est assurée par deux joints toriques 94 et 95. La partie centrale du couvercle 84 forme en outre une zone de recueil 91 du liquide épuré, qui se prolonge par une tubulure axiale 89 de sortie de ce liquide épuré.

Les figures Xa et Xb représentent un autre mode de réalisation d'un dispositif d'épuration 90 intégrant une membrane 101 de filtration tangentielle du liquide à épurer disposée en hélice autour d'une membrane microporeuse 102 d'épuration biospécifique. Cette membrane microporeuse 102 est une membrane plane elle-même enroulée autour d'un cylindre 103. Ces membranes sont contenues dans une enveloppe cylindrique 104 possédant latéralement une tubulure 96 d'entrée du liquide à traiter, une tubulure 97 de sortie du liquide concentré en éléments cellulaires.

Le dispositif d'épuration 90 comporte en outre une tubulure 98 de sortie du liquide épuré ainsi qu'une tubulure 99 permettant une prise de pression du liquide filtré à épurer.

Quel que soit le mode de réalisation choisi, le dispositif d'épuration objet de la présente invention permet de traiter un liquide dans un boitier unique successivement par filtration tangentielle puis immédiatement par filtration transversale.

Le liquide traité peut être du sang, ainsi que cela a été décrit, mais également tout autre liquide contenant des éléments cellulaires, que l'on souhaite éliminer avant d'assurer l'épuration biospécifique du liquide. Ainsi, dans le domaine des biotechnologies, il est possible d'assurer le traitement d'une suspension cellulaire, telle qu'une suspension de cellules transformées ou de bactéries, en retenant tout d'abord les cellules par filtration tangentielle le long de la membrane de filtration, puis en épurant le liquide par filtration transversale à travers la structure microporeuse traitée pour assurer l'épuration biospécifique du liquide, afin d'isoler par exemple des anticorps monoclonaux ou des protéines fabriquées par les bactéries.

## Revendications

1. Dispositif pour l'épuration biospécifique d'un liquide contenant des éléments cellulaires du type comprenant un boîtier muni de :
   – au moins une canalisation (11, 41, 57, 72, 85, 96) d'entrée du liquide à traiter,
   – au moins une canalisation (10, 12, 42, 58, 75, 86, 97) de sortie du liquide concentré en éléments cellulaires,
   – une canalisation (8, 44, 59, 81, 89) de sortie de la fraction liquide épurée,
   et à l'intérieur duquel est disposée au moins une membrane (2, 34, 67, 101) pour séparer par filtration tangentielle la fraction liquide des éléments cellulaires du liquide à traiter,
   caractérisé en ce que ledit boîtier comporte en outre au moins une membrane microporeuse (3, 35, 68, 102) pour l'épuration biospécifique par filtration transversale de la fraction liquide dudit liquide à traiter.

2. Dispositif selon la revendication 1, caractérisé en ce que ledit boîtier est constitué essentiellement par l'assemblage d'une plaque supérieure (5) et d'une plaque inférieure (4).

3. Dispositif selon l'une des revendications précédentes, caractérisé en ce qu'il comporte en outre une plaque intermédiaire (17) entre la membrane (2) pour séparer par filtration tangentielle la fraction liquide des éléments cellulaires du liquide à traiter et la structure microporeuse (3) pour l'épuration biospécifique.

4. Dispositif selon la revendication 3, caractérisé en ce que ladite plaque intermédiaire est munie sur chacune de ses deux faces principales d'un réseau de cannelures destinées à favoriser la circulation de la fraction liquide obtenue par filtration tangentielle du liquide à traiter.

5. Dispositif selon la revendication 1, caractérisé en ce que ledit boîtier est constitué essentiellement par une coquille cylindrique (33) fermée à chacune des deux extrémités par un couvercle (38, 39, 55, 56, 69, 70, 83, 84).

6. Dispositif selon l'une des revendications précédentes caractérisé en ce que la membrane microporeuse (3) est une membrane de type plan.

7. Dispositif selon l'une des revendications 1 à 5, caractérisé en ce qu'il comporte un empilement de membranes microporeuses de type plan disposées directement les unes au-dessus des autres.

8. Dispositif selon l'une des revendications précédentes, caractérisé en ce que ladite membrane pour séparer par filtration tangentielle la fraction liquide des éléments cellulaires du liquide à traiter est constituée par une membrane de type plan.

9. Dispositif selon la revendication 1, 5 ou 6, caractérisé en ce que ladite membrane pour séparer par filtration tangentielle la fraction liquide des éléments cellulaires du liquide à traiter est constituée par un ensemble de fibres creuses (34, 68).

10. Dispositif selon la revendication 9, caractérisé en ce que ladite membrane microporeuse pour l'épuration biospécifique est une membrane (35) de type plan enroulée autour de l'ensemble de fibres creuses (34).

11. Dispositif selon la revendication 9, caractérisé en ce que ladite membrane microporeuse pour l'épuration biospécifique est une membrane (35) de type plan enroulée sur elle-même et disposée à l'intérieur de l'ensemble de fibres creuses (34).

12. Dispositif selon les revendications 1 ou 5, caractérisé en ce que ladite membrane pour séparer par filtration tangentielle les éléments cellulaires du liquide à épurer est constituée par un faisceau interne (67) de fibres creuses et en ce que ladite membrane microporeuse pour l'épuration biospécifique est constituée par un faisceau externe (68) de fibres creuses.

13. Dispositif selon les revendications 1 ou 5, caractérisé en ce que ladite membrane pour séparer par filtration tangentielle les éléments cellulaires du liquide à épurer est constituée par un faisceau externe (67) de fibres creuses et en ce que ladite membrane microporeuse pour l'épuration biospécifique est constituée par un faisceau interne (68) de fibres creuses.

**Patentansprüche**

1. Vorrichtung für die biospezifische Reinigung einer Zellelemente enthaltenden Flüssitgkeit von der Art umfassend ein Gehäuse versehen mit:
– wenigstens einer Leitung (11,41,57,72,85,96) zum Zuführen der zu behandelnden Flüssigkeit,
– wenigstens einer Leitung (10,12,42,58,75,86,97) zum Abführen der an Zellelementen konzentrierten Flüssigkeit,
– einer Leitung (8,44,59,81,89) zum Abführen der gereinigten Flüssigkeitsfraktion,
und in dessem Inneren wenigstens eine Membran (2,34,67,101) angeordnet ist zum Trennen durch tangentiale Filtration der Flüssigkeitsfraktion der Zellelemente von der zu behandelnden Flüssigkeit,
dadurch gekennzeichnet daß das Gehäuse außerdem wenigstens eine mikroporöse Membran (3,35,68,102) umfaßt zur biospezifischen Reinigung durch transversale Filtration der Flüssigkeitsfraktion der zu behandelnden Flüssigkeit.

2. Vorrichtung nach Anspruch 1, dadurch gekennzeichnet, daß das Gehäuse im wesentlichen ausgebildet ist durch den Aufbau einer oberen Platte (5) und einer unteren Platte (4).

3. Vorrichtung nach einem der vorhergehenden Ansprüche, dadurch gekennzeichnet, daß sie außerdem umfaßt eine Zwischenplatte (17) zwischen der Membran (2) zum Trennen durch tangentiale Filtration der Flüssigkeitsfraktion der Zellelemente von der zu behandelnden Flüssigkeit und der mikroporösen Struktur (3) zur biospezifischen Reinigung.

4. Vorrichtung nach Anspruch 3, dadurch gekennzeichnet, daß die Zwischenplatte auf jeder seiner zwei Hauptflächen mit einem Netz von Rillen versehen ist, die zur Unterstützung der Zirkulation der Flüssigkeitsfraktion bestimmt sind, die durch tangentiale Filtration der zu behandelnden Flüssigkeit erhalten wird.

5. Vorrichtung nach Anspruch 1, dadurch gekennzeichnet, daß das Gehäuse im wesentlichen durch eine zylindrishe Kokille (33) ausgebildet ist, die an jedem der beiden Enden durch eine Abdeckung (38,39,55,56,69,70,83,84) verschlossen ist.

6. Vorrichtung nach einem der vorhergehenden ansprüche, dadurch gekennzeichnet, daß die mikroporöse Membran (3) eine Membran vom planaren Typ ist.

7. Vorrichtung nach einem der Ansprüche 1 bis 5, dadurch gekennzeichnet, daß die einen Stapel von mikroporösen Membranen vom planaren Typ umfaßt, die direkt übereinander angeordnet sind.

8. Vorrichtung nach einem der vorhergehenden Ansprüche, dadurch gekennzeichnet, daß die Membran zum Trennen durch tangentiale Filtration der Flüssigkeitsfraktion der Zellelemente von der zu behandelnden Flüssigkeit durch eine Membran vom planaren Typ ausgebildet ist.

9. Vorrichtung nach einem der Ansprüche 1,5 oder 6, dadurch gekennzeichnet, daß die Membran zum Trennen durch tangentiale Filtration der Flüssigkeitsfraktion der Zellelemente von der zu behandeladen Flüssigkeit durch einen Satz Hohlfasern (34,68) ausgebildet ist.

10. Vorrichtung nach Anspruch 9, dadurch gekennzeichnet, daß die mikroporöre Membran zum biospezifischen Reinigen eine Membran (35) vom planaren Typ ist, die um den Satz der Hohlfasern (34) gewickelt ist.

11. Vorrichtung nach Anspruch 9, dadurch gekennzeichnet, daß die mikroporöre Membran zum biospezifischen Reinigen eine Membran (35) vom plenaren Typ ist, die um sich gewickeit ist und im inneren des Satzes der Hohlfasern (34) angeordnet ist.

12. Vorrichtung nach Anspruch 1 oder 5, dadurch gekennzeichnet, daß die Membran zum Trennen durch tangentiale Filtration der Zellelemente von der zu reinigenden Flüssigkeot durch ein inneres Bündel (67) der Hohlfasern ausgebildet ist und daß die mikroporöre Membran zum biospezifischen Reinigen durch ein äußeres Bündel (68) der Hohlfaser ausgebildet ist.

13. Vorrichtung nach den Ansprüchen 1 oder 5, dadurch gekennzeichnet, daß die Membran zum Trennen durch tangentiale Filtration der Zellelemente von der zu reinlgenden Flüssigkeit durch ein äußeres Bündel (67) der Hohlfasern ausgebildet ist und daß die mikroporöse Membran zum biospezifischen Reinigen durch ein Inneres Bündel (68) der Hohlfasern ausgebildet ist.

**Claims**

1. A device for the biospecific purification of a liquid containing cellular elements of the type comprising a casing provided with:
   – at least one inlet duct (11, 41, 57, 72, 85, 96) of the liquid to be treated,
   – at least one outlet duct (10. 12, 42, 58, 75, 86, 97) for the liquid with a concentration of cellular elements,
   – an outlet duct (8, 44, 59, 81, 89) for the purified liquid fraction,
   and inside which at least one membrane (2, 34, 67, 101) is disposed to separate by tangential filtration the liquid fraction from the cellular elements of the liquid to be treated,
   characterized in that the said casing comprises, moreover, at least one microporous membrane (3, 35, 68, 102) for the biospecific purification by transverse filtration of the liquid fraction of the said liquid to be treated.

2. A device according to claim 1, characterized in that the said casing is essentially constituted by the assembly of an upper plate (5) and of a lower plate (4).

3. A device according to one of the preceding claims, characterized in that it comprises, moreover, an intermediate plate (17) between the membrane (2) for separating the liquid fraction by tangential filtration from the cellular elements of the liquid to be treated and the microporous structure (3) for the biospecific purification.

4. A device according to claim 3, characterized in that the said intermediate plate is provided on each of its two main faces with a network of grooves intended to promote the circulation of the liquid fraction obtained by targential filtration of the liquid to be treated.

5. A device according to claim 1, characterized in that the said casing is essentially constituted by a cylindrical shell (33) closed at each of its two ends by a cover (38, 39, 55, 56, 69, 70, 83, 84).

6. A device according to one of the preceding claims, characterized in that the microporous membrane (3) is a flat type membrane.

7. A device according to one of claims 1 to 5, characterized in that it comprises a stack of flat type microporous membranes disposed directly on top of one another.

8. A device according to one of the preceding claims, characterized in that the said membrane for separating the liquid fraction by tangential filtration from the cellular elements of the liquid to be treated is constituted by a flat type membrane.

9. A device according to claim 1, 5 or 6, characterieed in that the said membrane for separating the liquid fraction by tangential filtration from the cellular elements of the liquid to be treated is constituted by a set of hollow fibres (34, 68).

10. A device according to claim 9, characterized in that the said microporous membrane for the biospecific purification is a flat type membrane (35) wrapped around the set of hollow fibres (34).

11. A device according to claim 9, characterized is that the said microporous membrane for the biospecific purification is a flat type membrane (35) wrapped around itself and disposed inside the set of hollow fibres (34).

12. A device according to claims 1 or 5, characterized in that the said membrane for separating the cellular elements by tangential filtration from the liquid to be purified is constituted by an inner bundle (67) of hollow fibres and in that the said microporous membrane for the biospecific purification is constituted by an external bundle (68) of hollow fibres.

13. A device according to claims 1 or 5, characterized in that the said membrane for separating the cellular elements by tangential filtration from the liquid to be purified is constituted by an outer bundle (67) of hollow fibres, and in that the said microporous membrane for the biospecific purification is constituted by an inner bundle (68) of hollow fibres.

Figure I

Figure II

Figure III

Figure Ⅳ a

30

13

3

24

17

107

2

11

2

107

17

24

3

13

31

12

29

Figure IV b

Figure Ⅴ a

Figure $\overline{V}$ b

54 38 45 43 41

50

49

36

34

37

35

33

X X'

51

44

53 39 52 48 46 42

47

49

108
109
33

Figure VI c

37 34

35 33

36

Figure VI b

Figure VI a

Figure VII b

Figure VII c

Figure VII a

Figure VIII b

Figure VIII a

83  92  87  93  85

80

67

37

68

33

P  P'

33  67  68

37

Figure IX b

82

84 / 94 / 88 / 95 / 89 / 91 / 86

Figure IX a

Figure Xa

Figure Xb